# EUROPEAN PATENT SPECIFICATION

(11) **EP 0 652 974 B1**
(45) Date of publication and mention of the grant of the patent: **02.05.1997**
(21) Application number: 93917903.2
(22) Date of filing: 23.07.1993
(51) Int. Cl.: C12Q 1/68

(54) **DETECTION OF CRYPTOSPORIDIUM**
NACHWEISS VON CRYPTOSPORIDIUM
DETECTION DU CRYPTOSPORIDIUM

(30) Priority: 23.07.1992 GB 9215656
(43) Date of publication of application: 17.05.1995
(73) Proprietor: MINISTER OF AGRICULTURE, FISHERIES AND FOOD IN HER BRITANNIC MAJESTY'S GOV. OF THE U.K. OF GREAT BRITAIN AND NORTHERN IRELAND, London SW1A 2HH (GB)
(72) Inventor: WOODWARD, Martin John, Farnborough, Hampshire GU14 8NN (GB); WEBSTER, Katherine Anne, Dorking, Surrey RH4 3HT (GB)
(74) Representative: Lockwood, Peter Brian
(86) International application number: GB9301559
(87) International publication number: WO9402635

(56) References cited:
- 40TH ANNUAL MEETING OF THE AMERICAN SOCIETY OF TROPICAL MEDICINE AND HYGIENE, BOSTON, MASSACHUSETTS, USA, DECEMBER 1-5, 1991. AM J TROP MED HYG 45 (3 SUPPL.). 1991. 229. CODEN: AJTHAB ISSN: 0002-9637 13 - (C) FILE BIOSIS LAXER M A ET AL 'DETECTION OF ***CRYPTOSPORIDIUM*** -PARVUM DNA IN FIXED PARAFFIN-EMBEDDED TISSUE BY THE ***POLYMERASE*** ***CHAIN*** ***REACTION***.'
- AMERICAN JOURNAL OF TROPICAL MEDICINE AND HYGIENE vol. 45, no. 6 , 1991 pages 688 - 694 LAXER ET AL. 'DNA sequences for the specific detection of Cryptosporidium parvum by PCR'
- BIOCHIM BIOPHYS ACTA 1131 (3). 1992. 317-320. CAI J ET AL '***PCR*** CLONING AND NUCLEOTIDE SEQUENCE DETERMINATION OF THE 18S RRNA GENES AND INTERNAL TRANSCRIBED SPACER 1 OF THE PROTOZOAN PARASITES ***CRYPTOSPORIDIUM*** -PARVUM AND ***CRYPTOSPORIDIUM*** -MURIS.'

## Description

The present invention relates to a method for the detection and/or identification of organisms and/or nucleic acid sequences of Cryptosporidium parvum (C.parvum) or baileyi (C.baileyi) and to oligonucleotides and test kits containing them for use in said method.

Cryptosporidosis is a parasitic disease caused by protozoans of the coccidial genus Cryptosporidium. In the UK, the first human infections were reported to the Communicable Disease Surveillance Service in June 1983 and between then and 1989 reported cases had risen to 8,019 per annum.

The disease has an incubation period of 1-14 days with symptoms lasting for 7-60 days and pathogenicity demonstrated mainly in a syndrome of diarrhoea of newborns and in cases of severe and prolonged gastroenteritis in immunodeficient patients. However, in people with a normal immune response the vast majority of cases involve acute self-limiting diarrhoeal symptoms. There is no chemotherapy, only supportive treatment can be offered.

The following species and respective hosts have been determined by cross-transmission studies:

| | |
|---|---|
| C.parvum | mammals |
| C.muris | rodents (mice) |
| C.baileyi | avians |
| C.meleagridis | avians |
| C.crotali* | reptiles |
| C.nasorum* | fish |

| | |
|---|---|
| * = Not thought to occur in the UK. | |

Large numbers of oocysts have been recovered from waste, surface and recreational waters, but their origin is unknown. These oocysts have been known to survive for considerable periods in water and are unaffected by conventional water treatments (Smith et al (1989) Parasitology 99:323-7).

It is known that cryptosporidiosis is a zoonosis and several human outbreaks have implicated bovine slurry runoff into water sources as a focus of infection, but the importance of this route is not fully understood as currently there is no adequate test to distinguish between different oocysts (Chermetie et al (1988) Cryptosporidiosis: 2nd edition. Technical series number 5. Office International des epizooties. Paris).

The present invention provides an improved method for diagnosis of cryptosporidiosis, an important zoonosis involving farm animals, and further provides oligonucleotide probes and polymerase chain reaction primers specific to C.parvum and C.baileyi and still further provides probes and primers which differentiate between the two.

The invention provides a method of detecting and identifying C.parvum and baileyi specifically by identifying DNA of SEQ ID No 7 or 8, or subsequences having SEQ ID No 1, 2, 5 or 6 described herein, by use of hybridization probing and/or nucleotide sequence amplification, eg by polymerase chain reaction (PCR) or ligase chain reaction (LCR). Amplification of these sequences by the PCR or LCR provides direct indication of a positive result through observation of product formation of predicted size or confirmatory hybridization probing. The specific oligonucleotide probes and primers of the invention can be applied to suitably treated samples (eg. surface water faeces etc.) not only to detect but also to differentiate these cryptosporidia.

Probing will conveniently be carried out by using radiolabeled probes in Southern Blotting, but use of other probing techniques is also provided, eg. by tagging oligonucleotides complementary to the target sequence with a marker, eg. such as biotin or a fluorescent agent, and relating presence of bound marker with a positive result, eg. by using biotin tag as active agent in an ELISA in the known manner or detecting fluorescence.

The present invention particularly provides a method capable of the detection of C.parvum and C.baileyi oocysts in contaminated water, a task requiring extremely sensitive techniques. The specificity of the method of the present invention has been confirmed by testing for cross-reactivity with Giardia and Eimeria and other water-borne organisms and microbes.

Typically the specific sequence amplification primers of the invention are applied to crude preparations of oocysts, faecal or water samples, together with chain reaction reagents and the performance of the reaction is assessed under these conditions. Oocysts may be isolated by concentration from water according to the method of Musial and colleagues, and DNA released. Amplification using PCR or LCR can then be used to detect the presence of cryptosporidia and differential hybridization probing or further PCR or LCR used to speciate them.

A first aspect of the present invention provides a method for the detection and/or identification of C. parvum organisms, or polynucleic acid specific thereto, comprising determining the presence of the nucleic acid sequence SEQ ID No 1, or its complementary sequence SEQ ID No 2, in polynucleic acid present in or derived from a sample under investigation, and relating the presence thereof to presence of C.parvum or polynucleic acid thereof.

In a preferred embodiment of this aspect of the present invention the method comprises hybridization probing polynucleic acid present in or derived from a sample under investigation with an oligonucleotide that is capable of specific hybridization with sequences characteristic of the sequence SEQ ID No 1, or its complementary sequence SEQ ID No 2, and relating the occurence of hybridization with the presence of C. parvum or polynucleic acid thereof. Such hybridization may be carried out by any of the conventionally known methods, eg. by Southern Blotting or one of the tagging methods referred to above.

The term 'characteristic of' as used herein refers to sequences that have sufficient consecutive bases homologous with the sequence of interest to be considered statistically highly likely to be that sequence. Thus herein a 'characteristic sequence' is one identical to any 10 consecutive bases of the sequence of interest, in this case SEQ ID No 1 or 2. Preferred probes are of SEQ ID No 1 and/or 2.

The specificity of the probing is controlled in the known manner by selecting stringency conditions such that the chosen probe will not hybridize with sequences found in other organisms that are known to be present in or considered likely to be found in such samples. This can be determined by carrying out control experiments. Furthermore, probes can be confirmed as specific by comparing their complementary sequences to sequences found in other known organisms. Such comparison can be readily made using computer databases, such as EMBL or GENBANK, whereby rapid comparison of sequences can be made.

The present invention further provides polynucleotide probes suitable for this method, preferably comprising a polynucleotide of SEQ ID No1 and/or 2, said probe being optionally labelled in known manner, eg. by incorporation of a radioactive (eg. ³²P), chemical or biological label into its structure; most preferably consisting of these sequences.

In a preferred embodiment the present invention provides a method for the detection and/or identification of C.parvum organisms or nucleic acid specific thereto comprising use of specific sequence amplification, eg. the polymerase chain reaction or ligase chain reaction, to amplify SEQ ID No 1 and/or SEQ ID No 2 . The use of primers in the known manner may be made to target these sequences to provide an all or nothing amplification indicative of the presence of C. parvum.

Thus, for example, for the polymerase chain reaction, forward and reverse PCR primers having 5' end sequences SEQ ID No 3 and SEQ ID No 4 may be used, with or without 5' end non-hybridizing extensions: eg. consisting of these sequences or any other primers which specifically provide for PCR amplification of the C. parvum specific sequences SEQ ID No 1 and/or 2. It will be understood by those skilled in the art that for ligase chain reaction longer primers will be required, having a smaller gap between them on the target sequence.

In a second aspect the present invention provides a method for the detection or identification of C.parvum or C.baileyi organisms or polynucleic acid specific thereto, comprising determining the presence of the nucleic acid SEQ ID No 5 and/or 6, in polynucleic acid present in or derived from a sample under investigation, and relating the presence thereof to presence of one of these organisms or their polynucleic acid.

In a preferred embodiment of this aspect the method comprises hybridization probing polynucleic acid, present in or derived from a sample under investigation, with an oligonucleotide that is capable of specific hybridization with a sequence characteristic of sequences SEQ ID No 5 or the sequence complementary thereto, SEQ ID No 6, these being sequences of the Cryptosporidium genome in which SEQ ID No 1 and 2 are located in C. parvum; ie. bases 123 to 152 of SEQ ID 7 and the corresponding part of SEQ ID No 8 in the sequence listing attached. In the C. baileyi genome the SEQ ID No 1 and 2 are somewhat different and thus identifying sequences that consist of mainly of the characteristic parts of these is not indicative of that organism. Thus for detection of both of these species, or positive identification of C.baileyi alone, the aforesaid sequences SEQ ID 1 and/or 2 should not be used unless part of larger probes used under less stringent hybridization conditions.

In a further aspect of the invention the method determines the presence of a SEQ ID No 7 and/or 8, as referred to above, and relates that to the presence of C.parvum or C. baileyi and or polynucleic acid of either. Preferably the method comprises hybridization probing with a sequence capable of specifically hybridizing with sequnces characteristic of these, as defined above, again not using those having SEQ ID No 1 and/or 2 to positively identify C.baileyi.

Specific hybridization may be carried out in each case with hybridization conditions stringent enough to exclude interfering organisms and genomic DNA from other Cryptospridium organisms while being of low enough stringency to allow hybridization with both the parvum and baileyi sequences. The determination of such conditions will be readily made by those skilled in the art by use of simple control experiments and the protocols outlined in the examples provided herein.

The present invention further provides a method for detecting or identifying C.parvum or C.baileyi organisms or nucleic acid sequences specific thereto comprising
(a) mixing a sample suspected of comprising said organisms and/or nucleic acid sequences with polymerase chain reaction or ligase chain reaction reagents, and respective primers targeted to specifically amplify a polynucleotide sequence within SEQ ID No 7 and/or 8 comprising polynucleotides of SEQ ID No 5 and/or 6;
(b) subjecting the mixture to conditions under which amplification of any sequence comprising SEQ ID No 5 and/or 6 present will occur;
(c) relating the production of a polymerase chain reaction product corresponding in size to SEQ ID No 5 and/or 6 to the presence of C. parvum and/or C.bailyei organisms or nucleic acid sequences specific thereto.

Preferably the polynucleotide sequence amplified is the double stranded sequence found within SEQ ID No 7 and 8, shown between base 91 and 420 of SEQ ID No 7, or the equivalent sequence of C. baileyi wherein the sequences equivalent to SEQ ID No 1 and 2 are present. Thus the preferred polynucleic acid sequences amplified are those amplified by PCR primers of SEQ ID No 9 and 10 described in the sequence listing given herein.

Again, in order to ensure specific amplification of the target sequence it is convenient to determine the degree of specificity required of the primers to be selected by either using control experiments using DNA of organisms likely to be found in the samples to be tested as controls with which the selected primers should not initiate a polymerase chain reaction. In a further method of selecting primers the primer nucleotide sequence contemplated for use may be compared to known sequences on databases such a EMBL and Genbank where a finding of low homology with recorded sequences, particularly in the area of the primer 5' end sequence, indicates suitability for specific amplification.

Where verification of production of a PCR or LCR product or a native unamplified sequence as being one of C. parvum or C. baileyi is intended then probing may be carried out using a labelled probe specific for the sequences above eg. probes comprising any of the sequences SEQ ID No 1 to 8 or double stranded sequences containing these. Smaller probe sequences selected from the aforesaid sequences will also be derivable by the person skilled in the art by use of control experiments and/or databases referred to above, but it is generally recognised that sequences of complementary to 10 or more consecutive bases of the target polynucleic acid will be specific enough in binding for verification of presence of such sequences.

Preferably, where PCR is used, the primers should be of 6 or more bases long, more preferably 10 or more, eg. 25 bases and most preferably from 10 to 20 bases long.

Particularly preferred PCR primers found to be specific for amplification of DNA characteristic of SEQ ID No 7 and/or 8 for the purposes of identifying a sequence including SEQ ID No 5 and/or 6 against a background of other organisms have 5' end SEQ ID No 9 (forward primer) and SEQ ID No 10 (rearward primer).

Most preferably the primer sequences consist of these sequences. The present invention further provides such primers per se.

The unamplified or amplified polynucleotide sequence may be identified by hybridization probing using a suitably labelled oligonucleotide that is capable of specific hybridization with the sequence SEQ ID No 5 and/or 6 or larger sequences containing them, eg. SEQ ID No 7 and 8 or double stranded sequences made up of these.

Where it is required to determine the presence or identity of one of C.parvum or baileyi specifically they can be identified by use of a probe of for SEQ ID No 1 and/or 2 as described above; hybridization being indicative of C. parvum. The present invention further provides a method of determining the presence of C.parvum as opposed to C. baileyi by use PCR or LCR to amplify the sequences specific to both and then applying PCR, LCR or hybridization probing to the product to determine the presence or otherwise of SEQ ID No 1 and/or 2, and relating that to presence of one or other of C. parvum or baileyi.

It will be understood by those skilled in the art that when any of the methods outlined above are being applied to whole organisms, rather than to nucleic acids derived from them, it will be necessary to incorporate a procedure for liberating nucleic acids from said organism before initiating amplification chain reaction conditions.

The preferred forms of the present invention provide particular procedures whereby the sample to be tested is first suspended in a medium, eg. a buffer, and then either sonicated or subjected to freeze/thaw cycles in buffer containing a reducing agent, eg. dithiothreitol (DTT), in order to rupture the organisms, particularly their oocyst forms, and liberate the polynucleic acids. Particularly preferred are formats that isolate oocysts using antibodies to oocysts that have been tagged with metallic particles such that use of magnetic fields can recover individual oocyst/antibody complexes.

Using these preferred methods of the present invention it is possible to achieve detection or identification of Cryptosporidium organisms in oocyst form or otherwise. Preferably the sonication or freeze/thaw procedure is carried out in a medium which will form all or part of the PCR, LCR or probing medium, eg. the 1xTE medium referred to below, together with a reducing agent, eg 1-2% DTT in buffer. Sonication is conveniently carried out using about 11 µm peak to peak. Alternatively, for freeze/thaw procedure, the sample containers can be conveniently immersed in a cryogenic liquid such as liquid nitrogen. Other methods for disrupting cell or oocyst structures to liberate nucleic acids into the PCR or LCR probing medium will be known to the skilled person and may be expected to be applicable before initiating the PCR, LCR and/or probing procedures.

In a still further aspect of the present invention there are provided test kits comprising one or more probes and/or primers of the present invention as described herein above, in addition to these probes and primers per se.

The method, primers and probes of the present invention will now be described by way of illustration only by reference to the following protocols and examples. Further variants of the method, primers and probes falling within the scope of the invention will occur to the person skilled in the art in the light of these.

### EXAMPLE 1: POLYMERASE CHAIN REACTION DETECTION OF C. PARVUM NUCLEOTIDE SEOUENCES IN FORM OF ISOLATED DNA.

Primers SEQ ID No 9 and 10 were used in the following reaction mix/conditions to amplify an approximatetly 330 bp fragment from C.parvum and C.baileyi. These are 53F/53R in the priority document.

### Reaction mix (volume 50 µl)

| | |
|---|---|
| dntp,s | -250 µmoles of each |
| primers | -100 pmoles of each primer (SEQ ID 9 and 10) |
| glycerol | -5% v/v |
| Taq polymerase | -2.5 units |

1XTE (10mm Tris.cl pH 8; 1mm EDTA pH8)

| | |
|---|---|
| Buffer | 5.5 mm MgCl₂ |
| | 50mm KCl |
| | 10 mm Tris.cl pH 8.3 |

plus the target nucleic acid to be amplified in a volume of 1-10 µl.

### Reaction Conditions

| | |
|---|---|
| Initial denaturing step | 94°C for 3 minutes |
| Denature | 94°C for 90 seconds ) |
| Anneal | 50°C for 120 seconds) 40 cycles |
| Extend | 72°C for 120 seconds) |
| Final extension | 72°C for 5 minutes |

The sample is then run on a 2% horizontal agarose gel, stained with ethidium bromide and viewed under UV illumination whereby the method has been shown to detect 30 fg (30 x 10⁻¹⁵g) of relatively pure C. parvum DNA by comparison of the sample gel with those from positive and negative DNA controls. C.baileyi also gave an approximately 330 bp product using these conditions.

### EXAMPLE 2. PCR HYBRIDISATION PROBING.

The DNA on the stained gel produced in Example 1 was transferred onto a nylon filter (trademark-Hybond N) by Southern blotting, denatured and hybridised with a ³²P radiolabelled (multiprime labelling procedure) cloned C. parvum internal probe SEQ ID No 1. After washing off non-specifically bound probe, the filter is exposed to X-ray film (autoradiography) for 12 hours or 7 days. Detection limits are respectively: 12 hours 0.3 fg, 7 days 0.03 fg of C. parvum DNA. It will be realised that the hybridization probe might be used directly on the sample DNA without PCR but that this will necessarily reduce sensitivity. C. baileyi derived DNA did not give a positive result in the hybridization probing with SEQ ID No 1.

Negative controls. DNA from a number of other organisms has been tested and proven negative both by visual examination of the PCR product on a gel and after hybridisation. Cryptosporidium muris; Giardia lamblia; Tritrichomonas foetus; Candida albicans; Eimeria spp mixed ovine culture; Staphylococcus aureus; Escherichia coli; Streptococcus bovis; Baccilus sp.; Actinobacter sp.; Pseudomonas sp.; Enterobacter sp.; Haemonchus contortus; Trichostrongylus sp.; Ostertagia sp.; DNA of bovine, murine and avian origin.

### EXAMPLE 3. PREPARATION OF WHOLE OOCYSTS FOR PCR.

A rapid protocol was used for preparation of oocysts (the infective stage of Cryptosporidium) for PCR to avoid the long procedure associated with preparation of DNA.

1. The sample to be tested is resuspended in a solution of 1X TE (see above); 1% dithiothreitol (approx final volume is 100µl) 2. The oocysts are broken open by a) 3 cycles of sonication for 10 seconds (11µm peak to peak) with a 50 second rest, or b) 3 freeze/thaw cycles in liquid nitrogen. 3) The sample is then heated to 90°C for 20 minutes. 4) Debris is pelleted by centrifugation at 13000g for 5 minutes. 5) 1-10 µl of the supernatant is used as the template for PCR.

A series of oocyst concentrations were used to determine the detection limits of the reaction. For C.parvum oocysts detection limits are: 2000 oocysts by visual examination of gel of PCR product; 20 oocysts following probing and autoradiography for 7 days.

### Cross-reactivity with other isolates.

4 bovine isolates of C.parvum obtained from different areas of the UK were examined and the region encoded by the SEQ ID No 5 and 6 is conserved in each of these isolates.

### EXAMPLE 4: APPLICATION OF METHOD TO HUMAN AND ANIMAL FAECAL SAMPLES.

Human faecal samples were obtained from 5 patients from three towns in Scotland; animal faecal samples were obtained from 15 sites in England.

Human faeces: each sample was diluted 1/10 and 1/100 in 1xTE/2%DTT buffer, freeze thawed in liquid nitrogen 90°C five times, heated to 90°C for 20 minutes, debris pelleted by centrifugation at 13,000g for 5 minutes and the supernatant used as template for PCR. The PCR method of the present invention was compared to A/P staining.

### Results:

| Sample | A/P STAIN | PCR(1/10) | PCR(1/100) |
|---|---|---|---|
| 1 | 2+ | + | + |
| 2 | - | + | + |
| 3 | 1+ | - | + |
| 4 | 2+ | + | + |
| 5 | 2+ | - | + |

The results were obtained when the tests were carried out on three separate occasions, and these and other results suggest that 1/100 is the optimal dilution. Tests were carried out on animal faeces as above using 1/100 dilution but using the SEQ ID No 7 as a probe to determine PCR product identity:

### Results:

| Host | A/P STAIN | PCR(1/100)+SEQ ID No 7 |
|---|---|---|
| Bovine | - | - |
| " | 2+ | + |
| " | 2+ | + |
| " | 3+ | + |
| " | + | + |
| " | - | - |
| " | +/- | - |
| Cervine | 4+ | + |
| Bovine | 3+ | + |
| " | 3+ | + |
| " | + | + |
| Ovine | - | + |
| Bovine | - | + |
| " | - | - |
| " | 3+ | + |
| - = negative + = 1 or 2 oocysts per slide 2+= 1 oocyst per field 3+= 2-5 oocysts per field 4+= 5-10 oocysts per field | | |

### SEQUENCE LISTING

(1) GENERAL INFORMATION:
   (i) APPLICANT:
      (A) NAME: THE MINISTER OF AGRICULTURE FISHERIES AND FOOD IN HER BRITA
      (B) STREET: WHITEHALL PLACE
      (C) CITY: LONDON
      (E) COUNTRY: UNITED KINGDOM (GB)
      (F) POSTAL CODE (ZIP): SW1A 2HH
   (ii) TITLE OF INVENTION: DETECTION OF CRYPTOSPORIDIUM (iii) NUMBER OF SEQUENCES: 10
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS (D) SOFTWARE:
         PatentIn Release £1.0, Version £1.25 (EPO)
      (vi) PRIOR APPLICATION DATA:
         (A) APPLICATION NUMBER: GB 9215656.1 (B) FILING DATE: 23-JUL-1992
(2) INFORMATION FOR SEQ ID NO: 1:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 1:
   ACGTAGCGCC GGACGACAGC AGCAGCGCGT
(2) INFORMATION FOR SEQ ID NO: 2:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 2:
   ACGCGCTGCT GCTGTCGTCC GGCGCTACGT 30
(2) INFORMATION FOR SEQ ID NO: 3:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 3:
   ACGTAGCGCC GGACG 15
(2) INFORMATION FOR SEQ ID NO: 4:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 4:
[2) INFORMATION FOR SEQ ID NO: 5:
   (i) SEQUENCE CHARACTERISTICS:
      (A) LENGTH: 240 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 5:
(2) INFORMATION FOR SEQ ID NO: 6:
   (i) SEQUENCE CHARACTERISTICS:
      (A) LENGTH: 240 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 6:
   ATTGTCTTTA AATATATATT ATTACTTAGC TGTTCAAAAT TATCTATGAA ATCTTCTCCG 60
   ATCTGTTTCT TGCAATGATA TTTCAATTGT AGTAATAATG ATCTGGCCGG ATTTTTCGGC 120
   TGTGTGGCCG AACAACGGTC CGGCAGCGCT GGTTTCGCCT TCAAGTCGAT GCAGCCGTGG 180
   CCGGTGCCGA CCATCAACAC GCGCTGCTGC TGTCGTCCGG CGCTACGTTG ACGGTTTCGC 240
(2) INFORMATION FOR SEQ ID NO: 7:
   (i) SEQUENCE CHARACTERISTICS:
      (A) LENGTH: 523 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 7:
   GATCTCTAGC CAACGTTGAC CACCTTCTTT TAATAGCCAA GTTAAAATAG CTCTGAATAT 60
   TCTTGGAGCA GATTGAGCAA TAAAGTGAAG ATCTTCACGC AGTGCGTGGT GCGAAACCGT 120
   CAACGTAGCG CCGGACGACA GCAGCAGCGC GTGTTGATGG TCGGCACCGG CCACGGCTGC 180
   ATCGACTTGA AGGCGAAACC AGCGCTGCCG GACCGTTGTT CGGCCACACA GCCGAAAAAT 240
   CCGGCCAGAT CATTATTACT ACAATTGAAA TATCATTGCA AGAAACAGAT CGGAGAAGAT 300
   TTCATAGATA ATTTTGAACA GCTAAGTAAT AATATATATT TAAAGACAAT TTCTAGCCAT 360
   TTAAAAAAGG TTTTTAGAAG GGATCCGGGC AAGTCCGGCG TCGACATCTA CCGGCTGATG 420
   AAGTTCCAGC GCTCGAACCA GTCGACCTGC ATCAACACGT CCGCTGGTCA AGGTGGGCGA 480
   CCGCATCGAG AAGGGCGACA TCATCGCTGA CGGTCCTTCG ACC 523
(2) INFORMATION FOR SEQ ID NO: 8:
   (i) SEQUENCE CHARACTERISTICS:
      (A) LENGTH: 523 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM
      (B) STRAIN: PARVUM
   (xi) SEQUENCE DESCRIPTION: SEQ ID NO: 8:
(2) INFORMATION FOR SEQ ID NO: 9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM
      (B) STRAIN: PARVUM
   (xi) SEQUENCE DESCRIPTION: SEQ ID NO: 9:
   ATCTTCACGC AGTGCGTGGT    20
(2) INFORMATION FOR SEQ ID NO: 10:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iii) ANTI-SENSE: NO
   (vi) ORIGINAL SOURCE:
      (A) ORGANISM: CRYPTOSPORIDIUM PARVUM
   (xi) SEQUENCE DESCRIPTION: SEQ ID NO: 10:
   CATCAGCCGG TAGATGTCGA                                                     20

## Claims

1. A method for the detection and/or identification of C. parvum organisms, or nucleic acid sequences specific thereto, comprising determining the presence of the nucleic acid sequence SEQ ID No 1 and/or 2, in polynucleic acid present in or derived from a sample under investigation, and relating the presence thereof to the presence of C. parvum or polynucleic acid thereof.

2. A method for the detection and/or identification of C. parvum organisms, or nucleic acid sequences specific thereto, comprising hybridization probing polynucleic acid present in or derived from a sample under investigation with an oligonucleotide that is capable of specific hybridization with sequences chraracteristic of SEQ ID No 1 and/or 2 and relating the occurence of hybridization with the presence of C. parvum or polynucleic acid thereof.

3. A oligonucleotide hybridization probe comprising 10 or more consecutive bases of the sequences SEQ ID No 1 or 2.

4. A method for the detection and/or identification of C.parvum organisms or nucleic acid sequences specific thereto, comprising use of specific sequence amplification to amplify any SEQ ID No 1 and/or 2 present in a sample, or polynucleic acid derived therefrom, and relating the production of product to the presence of C.parvum or said nucleic acid.

5. A method as claimed in claim 4 wherein the specific sequence amplification is carried out using the polymerase chain reaction.

6. A method as claimed in claim 5 wherein the forward and reverse PCR primers have 5' end sequences of SEQ ID No 3 and 4 respectively.

7. A polymerase chain reaction primer capable of acting as a primer for amplifying SEQ ID No 1 and/or 2 and comprising an oligonucleotide of 5' end sequence of sequence SEQ ID No 3 or 4.

8. A polymerase chain reaction primer having an oligonucleotide sequence of SEQ ID No 3 or 4.

9. A method for the detection and/or identification of C.parvum or C.baileyi organisms, or nucleic acid sequences specific thereto, comprising determining the presence of the C. parvum nucleic acid sequence SEQ ID No 5 and/or 6, or the equivalent sequence present in C. baileyi, in polynucleic acid present in or derived from a sample under investigation, and relating the presence thereof to presence of one of these organisms or their polynucleic acid.

10. A method for the detection and/or identification of C. parvum or C. baileyi organisms, or nucleic acid sequences specific thereto, comprising hybridization probing polynucleic acid, present in or derived from a sample under investigation, with an oligonucleotide or polynucleotide probe that is capable of specific hybridization with a polynucleic acid sequence characteristic of polynucleic acid comprising a sequence SEQ ID No 5 and/or 6, other than SEQ ID No 1 and/or 2, and relating the occurrence of hybridization to the presence of said organisms or polynucleic acid.

11. A method as claimed in claim 10 wherein the probe is of SEQ ID No 5 and/or 6.

12. A method for the detection and/or identification of C. parvum or C. baileyi organisms, or nucleic acid sequences specific thereto, comprising determining the presence of the C.parvum nucleic acid SEQ ID No 7 and/or 8, or the equivalent sequence present in C. bailyei, in polynucleic acid present in or derived from a sample, and relating that to the presence of said organisms or nucleic acid.

13. A method as claimed in claim 12 wherein the polynucleic acid is probed with an oligonucleotiode or polynucleotide capable of specifically hybridizing with a sequence characteristic of SEQ ID No 7 and/or 8, or the equivalent sequences found in C. baileyi, and the occurence of hybridization is related to the presence of said organisms or nucleic acid.

14. An oligonucleotide or polynucleotide probe comprising any 10 or more consecutive bases of SEQ ID No 7 and/or 8, and not being of SEQ ID No 1 and/or 2.

15. A polynucleotide probe of SEQ ID No 7 and/or 8.

16. A method for the detection and/or or identification of C.parvum or C.baileyi organisms or nucleic acid sequences specific thereto comprising
(a) mixing a sample suspected of comprising said organisms and/or nucleic acid sequences with specific sequence amplification reaction reagents and primers for said amplification, said primers being targeted to specifically amplify a polynucleotide sequence comprising polynucleotides of SEQ ID No 5 and/or 6, or the equivalent sequence present in C. baileyi;
(b) subjecting the mixture to conditions under which the amplification of any sequence comprising SEQ ID No 5 and/or 6 present will occur;
(c) relating the production of any amplification product to the presence of C. parvum or C. baileyi organisms or nucleic acid sequences specific thereto.

17. A method as claimed in claim 16 wherein the specific sequence amplification reaction is the polymerase chain reaction and the primers used have 5' end sequences SEQ ID No 9 and 10.

18. A method as claimed in claim 17 wherein the primer sequences consist of sequences SEQ ID No 9 and 10.

19. A method as claimed in any one of claims 16, 17 or18 wherein the product polynucleotide is identified by application of a specific sequence amplification reaction to the product using primers targeted at SEQ ID No 1 and/or 2 and relating production of further product to the presence of C. parvum organisms or polynucleic acid and lack of further product to presence of C. baileyi organisms or polynucleic acid.

20. A method as claimed in claim 16, 17 or 18 wherein the product polynucleotide is identified by means of hybridization probing using a hybridization probe comprising sequences as claimed in claim 3, 14 or 15.

21. A method as claimed in claim 20 wherein the hybridization probe is specific for SEQ ID No 1 and/or 2, but not for the equivalent sequence of C. baileyi, and occurrence of specific hybridization is related to the presence of C. parvum or its specific polynucleic acids.

22. A polymerase chain reaction primer consisting of an oligonucleotide having a 5' end sequence of SEQ ID No 3, 4. 9 or 10.

23. A primer as claimed in claim 22 consisting of an oligonucleotide of SEQ ID No 3, 4, 9 or 10.

24. A method as claimed in any one of the preceding method claims incorporating a procedure for liberating nucleic acids from said organism before initiating amplification chain reaction conditions.

25. A method as claimed in claim 24 wherein the sample to be tested is either sonicated or subjected to freeze/thaw cycles in order to rupture the organisms and liberate the nucleic acids.

26. A method as claimed in claim 24 or 25 wherein the organisms are first suspended in a medium.

27. A method as claimed in any on of claims 24 to 26 wherein the organisms are in the form of oocysts.

28. A probe as claimed in claim 3, 14 or 15 wherein the oligonucleotide is labelled by incorporation of a radioactive, chemical or biological label into its structure.

29. A diagnostic kit comprising one or more probes or primers as claimed in any one of claims 3, 7, 8, 14, 15, 22, 23 or 28.

30. A diagnostic kit as claimed in claim 29 further comprising metal tagged antibodies to Crystosporidium oocysts.

## Patentansprüche

1. Verfahren zur Erfassung und/oder Identifizierung von Organismen der Species *Cryptosporidium parvum* oder von für diese Species spezifischen Nucleinsäuresequenzen durch
Ermittlung des Vorliegens der Nucleinsäuresequenz SEQ ID Nr. 1 und/oder Nr. 2 in Polynucleotiden, die in einer untersuchten Probe vorliegen oder davon abgeleitet sind,
und
Korrelierung des Vorliegens solcher Nucleinsäuresequenzen mit dem Vorliegen von *C. parvum* oder Polynucleotiden davon.

2. Verfahren zur Erfassung und/oder Identifizierung von Organismen der Species *C. parvum* oder von für diese Species spezifischen Nucleinsäuresequenzen durch Hybridisierung von in einer untersuchten Probe vorliegenden oder davon abgeleiteten Polynucleotiden mit einem Oligonucleotid als Sonde, das zur spezifischen Hybridisierung mit Sequenzen befähigt ist, die für die Nucleinsäuresequenz SEQ ID Nr. 1 oder Nr. 2 charakteristisch sind,
und
Korrelierung des Auftretens einer Hybridisierung mit dem Vorliegen von *C. parvum* oder Polynucleotiden davon.

3. Oligonucleotid-Hybridisierungssonde, die 10 oder mehr aufeinanderfolgende Basen der Nucleinsäuresequenzen SEQ ID Nr. 1 oder Nr. 2 enthält.

4. Verfahren zur Erfassung und/oder Identifizierung von Organismen der Species *C. parvum* oder von für diese Species spezifischen Nucleinsäuresequenzen durch spezifische Sequenzamplifikation zur Amplifizierung von Nucleinsäuresequenzen SEQ ID Nr. 1 und/oder Nr. 2, die in einer Probe oder einem davon abgeleiteten Polynucleotid vorliegen,
und
Korrelierung der Entstehung von Amplifikationsprodukt mit dem Vorliegen von *C. parvum* oder des Polynucleotids.

5. Verfahren nach Anspruch 4, wobei die spezifische Sequenzamplifikation unter Anwendung der Polymerase-Kettenreaktion durchgeführt wird.

6. Verfahren nach Anspruch 5, wobei der Vorwärts- und der Revers-Primer für die Polymerase-Kettenreaktion am 5'-Ende die Nucleinsäuresequenz SEQ ID Nr. 3 bzw. 4 aufweisen.

7. Primer für die Polymerase-Kettenreaktion, der befähigt ist, als Primer zur Amplifikation der Nucleinsäuresequenz SEQ ID Nr. 1 und/oder Nr. 2 zu wirken und ein Oligonucleotid umfaßt, dessen 5'-Ende die Nucleinsäuresequenz SEQ ID Nr. 3 oder Nr. 4 aufweist.

8. Primer für die Polymerase-Kettenreaktion, der die Oligonucleotidsequenz SEQ ID Nr. 3 oder Nr. 4 aufweist.

9. Verfahren zur Erfassung und/oder Identifizierung von Organismen der Species *C. parvum* oder *C. baileyi* oder von für diese Species spezifischen Nucleinsäuresequenzen durch
Ermittlung des Vorliegens der Nucleinsäuresequenz SEQ ID Nr. 5 und/oder Nr. 6 von *C. parvum* oder der äquivalenten Nucleinsäuresequenz, die in *C. baileyi* vorliegt, in Polynucleotiden, die in einer untersuchten Probe vorliegen oder davon abgeleitet sind,
und
Korrelierung des Vorliegens solcher Nucleinsäuresequenzen mit dem Vorliegen eines dieser Organismen oder eines Polynucleotids davon.

10. Verfahren zur Erfassung und/oder Identifizierung von Organismen der Species *C. parvum* oder *C. baileyi* oder von für diese Species spezifischen Nucleinsäuresequenzen durch
Hybridisierung von in einer untersuchten Probe vorliegenden oder davon abgeleiteten Polynucleotiden mit einem Oligonucleotid oder Polynucleotid als Sonde, das zur spezifischen Hybridisierung mit Polynucleotidsequenzen befähigt ist, die für ein Polynucleotid charakteristisch ist, das die Nucleinsäuresequenz SEQ ID Nr. 5 und/oder Nr. 6 aufweist und von SEQ ID Nr. 1 und/oder Nr. 2 verschieden ist,
und
Korrelierung des Auftretens einer Hybridisierung mit dem Vorliegen dieser Organismen oder von Polynucleotiden davon.

11. Verfahren nach Anspruch 10, wobei die Sonde die Nucleinsäuresequenz SEQ ID Nr. 5 und/oder Nr. 6 aufweist.

12. Verfahren zum Nachweis und/oder zur Identifizierung von Organismen der Species *C. parvum* oder *C. baileyi* oder von für diese Species spezifischen Nucleinsäuresequenzen durch
Ermittlung des Vorliegens der Nucleinsäuresequenz SEQ ID Nr. 7 und/oder Nr. 8 von *C. parvum* oder der äquivalenten Nucleinsäuresequenz, die in *C. baileyi* vorliegt, in Polynucleotiden, die in einer untersuchten Probe vorliegen oder davon abgeleitet sind,
und
Korrelierung des Vorliegens solcher Nucleinsäuresequenzen mit dem Vorliegen dieser Organismen oder Nucleinsäuren.

13. Verfahren nach Anspruch 12, wobei die Nucleinsäure mit einem Oligonucleotid oder Polynucleotid hybridisiert wird, das zur spezifischen Hybridisierung mit einer
Nucleinsäuresequenz, die für die Nucleinsäuresequenz SEQ ID Nr. 7 und/oder Nr. 8 charakteristisch ist, oder den äquivalenten Nucleinsäuresequenzen, die in *C. baileyi* vorliegen, befähigt ist,
und
Korrelierung des Auftretens einer Hybridisierung mit dem Vorliegen dieser Organismen oder dieser Nucleinsäuren.

14. Oligonucleotid- oder Polynucleotid-Sonden, die 10 oder mehr aufeinanderfolgende Basen der Nucleinsäuresequenzen SEQ ID Nr. 7 und/oder Nr. 8 enthalten und nicht die Sequenz SEQ ID Nr. 1 und/oder Nr. 2 aufweisen.

15. Polynucleotid-Sonde mit der Nucleinsäuresequenz SEQ ID Nr. 7 und/oder Nr. 8.

16. Verfahren zur Erfassung und/oder Identifizierung von Organismen der Species *C. parvum* oder *C. baileyi* oder dafür spezifischen Nucleinsäuresequenzen, das folgende Schritte umfaßt:
(a) Mischen einer Probe, von der vermutet wird, daß sie diese Organismen und/oder Nucleinsäuresequenzen enthält, mit Reagentien zur spezifischen Sequenzamplifikation und mit Primern hierfür, die darauf gerichtet sind, daß eine Polynucleotidsequenz, die Polynucleotide mit der Nucleinsäuresequenz SEQ ID Nr. 5 und/oder Nr. 6 umfaßt, oder die in *C. baileyi* vorliegende äquivalente Nucleinsäuresequenz spezifisch amplifiziert wird,
(b) Einwirkenlassen von Bedingungen auf das Gemisch, unter denen die Amplifikation beliebiger vorliegender Nucleinsäuresequenzen, in welchen die Nucleinsäuresequenzen SEQ ID Nr. 5 und/oder Nr. 6 enthalten sind, erfolgt
und
(c) Korrelierung der Entstehung eines Amplifikationsprodukts mit dem Vorliegen von Organismen der Species *C. parvum* oder *C. baileyi* oder hierfür spezifischen Nucleinsäuresequenzen.

17. Verfahren nach Anspruch 16, wobei die spezifische Sequenzamplifikation durch Polymerase-Kettenreaktion durchgeführt wird und die verwendeten Primer am 5'-Ende die Sequenzen SEQ ID Nr. 9 bzw. Nr. 10 aufweisen.

18. Verfahren nach Anspruch 17, wobei die Primersequenzen aus den Sequenzen SEQ ID Nr. 9 bzw. Nr. 10 bestehen.

19. Verfahren nach einem der Ansprüche 16 bis 18, wobei das Polynucleotid des Amplifikationsprodukts durch Anwendung einer spezifischen Sequenzamplifikationsreaktion auf dieses Produkt identifiziert wird, wobei Primer verwendet werden, die auf die Nucleinsäuresequenz SEQ ID Nr. 1 und/oder Nr. 2 gerichtet sind und die Entstehung eines weiteren Amplifikationsprodukts mit dem Vorliegen von Organismen der Species *C. parvum* oder entsprechender Polynucleotide und die Nichtentstehung eines weiteren Amplifikationsprodukts mit dem Vorliegen von Organismen der Species *C. baileyi* oder entsprechender Polynucleotide korreliert wird.

20. Verfahren nach einem der Ansprüche 16 bis 18, wobei das Polynucleotid des Amplifikationsprodukts durch Hybridisierung mit einer Sonde identifiziert wird, wobei eine Hybridierungssonde verwendet wird, die Sequenzen gemäß den Ansprüchen 3, 14 oder 15 aufweist.

21. Verfahren nach Anspruch 20, wobei die Hybridisierungssonde für die Nucleinsäuresequenz SEQ ID Nr. 1 und/oder Nr. 2, jedoch nicht für die äquivalente Nucleinsäuresequenz von *C. baileyi* spezifisch ist und das Auftreten einer spezifischen Hybridisierung mit dem Vorliegen von *C. parvum* oder dafür spezifischen Polynucleotiden korreliert wird.

22. Primer für die Polymerase-Kettenreaktion, der aus einem Oligonucleotid besteht, das am 5'-Ende die Nucleinsäuresequenz SEQ ID Nr. 3, Nr. 4, Nr. 9 oder Nr. 10 aufweist.

23. Primer nach Anspruch 22, der aus einem Oligonucleotid mit der Nucleinsäuresequenz SEQ ID Nr. 3, Nr. 4, Nr. 9 oder Nr. 10 besteht.

24. Verfahren nach einem der vorhergehenden Verfahrensansprüche, das ein Verfahren zur Freisetzung von Nucleinsäuren aus den betreffenden Organismen vor der Initiierung der Bedingungen für die Amplifikation durch Kettenreaktion beinhaltet.

25. Verfahren nach Anspruch 24, wobei die zu testende Probe zum Aufbrechen der Organismen und zur Freisetzung der Nucleinsäuren entweder beschallt oder Einfrier-/Auftau-Zyklen unterzogen wird.

26. Verfahren nach Anspruch 24 oder 25, wobei die Organismen zunächst in einem Medium suspendiert werden.

27. Verfahren nach einem der Ansprüche 24 bis 26, wobei die Organismen in Form von Oocysten vorliegen.

28. Sonde nach Anspruch 3, 14 oder 15, wobei das Oligonucleotid durch Einführung einer radioaktiven, chemischen oder biologischen Markierung in seine Struktur markiert ist.

29. Diagnosekit, der eine oder mehrere Sonden oder Primer nach einem der Ansprüche 3, 7, 8, 14, 15, 22, 23 und 28 enthält.

30. Diagnosekit nach Anspruch 29, der ferner metallmarkierte Antikörper gegen *Cryptosporidium*-Oocysten enthält.

## Revendications

1. Méthode de détection et/ou d'identification d'organismes de l'espèce C. parvum ou de séquences d'acides nucléiques spécifiques de ces organismes, qui comprend les étapes consistant à déterminer la présence de la séquence SEQ ID N° 1 et/ou 2 d'acides nucléiques dans l'acide polynucléique présent dans un échantillon soumis à l'examen ou dérivé de cet échantillon, et l'établissement du rapport entre sa présence et la présence de C.parvum ou de son acide polynucléique.

2. Méthode de détection et/ou d'identification d'organismes de l'espèce C.parvum ou de séquences d'acides nucléiques spécifiques de ces organismes, qui comprend les étapes consistant à sonder par hybridation l'acide polynucléique, présent dans un échantillon soumis à l'examen ou dérivé de cet échantillon, avec un oligonucléotide qui est capable d'hybridation spécifique avec des séquences caractéristiques de SEQ ID N° 1 et/ou 2 et à établir un rapport entre l'apparition de l'hybridation et la présence de C. parvum ou de son acide polynucléique.

3. Sonde d'hybridation d'oligonucléotide, comprenant 10 ou plus de 10 bases consécutives des séquences SEQ ID N° 1 ou 2.

4. Méthode de détection et/ou d'identification d'organismes de l'espèce C. parvum ou de séquences d'acides nucléiques spécifiques de ces organismes, qui comprend les étapes consistant à utiliser une amplification de séquence spécifique pour amplifier toute séquence SEQ ID N° 1 et/ou 2 présente dans un échantillon ou un acide polynucléique qui en est dérivé et à établir le rapport entre l'obtention du produit et la présence de C. parvum ou de l'acide nucléique en question.

5. Méthode suivant la revendication 4, dans laquelle on effectue l'amplification de séquence spécifique en utilisant la réaction de polymérisation en chaîne.

6. Méthode suivant la revendication 5, dans laquelle les amorces directe et inverse de PCR ont des séquences respectives d'extrémité 5' de SEQ ID N° 3 et 4.

7. Amorce pour réaction de polymérisation en chaîne capable d'agir comme une amorce pour l'amplification de SEQ ID N° 1 et/ou 2 et comprenant un oligonucléotide à séquence d'extrémité 5' de séquence SEQ ID N° 3 ou 4.

8. Amorce pour réaction de polymérisation en chaîne ayant une séquence d'oligonucléotide SEQ ID N° 3 ou 4.

9. Méthode de détection et/ou d'identification d'organismes de l'espèce C. parvum ou C. baileyi ou de séquences d'acides nucléiques qui en sont spécifiques, comprenant la détermination de la présence de la séquence SEQ ID N° 5 et/ou 6 d'acides nucléiques de C. parvum ou de la séquence équivalente existant dans C. baileyi dans l'acide polynucléique présent dans un échantillon, ou dérivé d'un échantillon, soumis à l'examen, et l'établissement du rapport entre la présence de la séquence et la présence de l'un de ces organismes ou de leur acide polynucléique.

10. Méthode de détection et/ou d'identification d'organismes de l'espèce C. parvum ou C. baileyi, ou de séquences d'acides nucléiques qui en sont spécifiques, comprenant le sondage par hybridation d'acide polynucléique, présent dans un échantillon soumis à l'examen ou dérivé de cet échantillon, avec une sonde oligonucléotidique ou polynucléotidique qui est capable d'hybridation spécifique avec une séquence d'acide polynucléique caractéristique de l'acide polynucléique comprenant une séquence SEQ ID N° 5 et/ou 6 autre que SEQ ID N° 1 et/ou 2 et l'établissement du rapport entre l'apparition de l'hybridation et la présence des organismes ou de l'acide polynucléique en question.

11. Méthode suivant la revendicaiton 10, dans laquelle la sonde est de séquence SEQ ID N° 5 et/ou 6.

12. Méthode de détection et/ou d'identification d'organismes de l'espèce C. parvum ou C. baileyi ou de séquences d'acides nucléiques qui en sont spécifiques, comprenant le détermination de la présence de la séquence SEQ ID N° 7 et/ou 8 d'acides nucléiques de C. parvum ou de la séquence équivalente présente dans C. baileyi, dans l'acide polynucléique présent dans un échantillon ou dérivé d'un échantillon et l'établissement du rapport entre cette présence et la présence des organismes ou de l'acide nucléique en question.

13. Méthode suivant la revendication 12, dans laquelle l'acide polynucléique est sondé avec un oligonucléotide ou un polynucléotide susceptible d'hybridation spécifique avec une séquence caractéristique de SEQ ID N° 7 et/ou 8 ou les séquences équivalentes observées dans C. baileyi, et l'existence de l'hybridation est rapportée à la présence des organismes ou de l'acide nucléique en question.

14. Sonde oligonucléotidique ou polynucléotidique comprenant 10 ou plus de 10 bases consécutives quelconques de SEQ ID N° 7 et/ou 8 et n'étant pas de SEQ ID N° 1 et/ou 2.

15. Sonde polynucléotidique de SEQ ID N° 7 et/ou 8.

16. Méthode de détection et/ou d'identification d'organismes de l'espèce C. parvum ou C. baileyi ou de séquences d'acides nucléiques spécifiques de ces organismes, qui comprend les étapes consistant
(a) à mélanger un échantillon suspecté de contenir ces organismes et/ou ces séquences d'acides nucléiques avec des réactifs pour réaction d'amplification de séquence spécifique et des amorces pour cette amplification, ces amorces ayant pour objectif d'amplifier spécifiquement une séquence polynucléotidique comprenant des polynucléotides de SEQ ID N°5 et/ou 6 ou la séquence équivalente présente dans C. baileyi ;
(b) à exposer le mélange à des conditions dans lesquelles l'amplification de toute séquence présente comprenant SEQ ID N° 5 et/ou 6 a lieu ;
(c) à établir la relation entre l'obtention de tout produit d'amplification et la présence d'organismes de l'espèce C. parvum ou C. baileyi ou des séquences d'acides nucléiques qui en sont spécifiques.

17. Méthode suivant la revendication 16, dans laquelle la réaction d'amplification de séquence spécifique est la réaction de polymérisation en chaîne et les amorces utilisées ont des séquences d'extrémité 5' de SEQ ID N° 9 et 10.

18. Méthode suivant la revendication 17, dans laquelle les séquences des amorces consistent en séquences SEQ ID N° 9 et 10.

19. Méthode suivant l'une quelconque des revendications 16, 17 ou 18, dans laquelle le polynucléotide formé comme produit est identifié par l'exposition à une réaction d'amplification de séquence spécifique du produit en utilisant des amorces ciblées à SEQ ID N° 1 et/ou 2 et l'établissement du rapport entre la formation d'autre produit et la présence d'organismes de l'espèce C. parvum ou de leur acide polynucléique, et entre l'absence d'autre produit et la présence d'organismes de l'espèce C. baileyi ou de leur acide polynucléique.

20. Méthode suivant la revendication 16, 17 ou 18, dans laquelle le polynucléotide formé comme produit est identifié au moyen du sondage par hybridation utilisant une sonde d'hybridation comprenant des séquences suivant les revendications 3, 14 ou 15.

21. Méthode suivant la revendication 20, dans laquelle la sonde d'hybridation est spécifique de SEQ ID N° 1 et/ou 2 mais non de la séquence équivalente de C. baileyi, et l'apparition d'une hybridation spécifique est liée à la présence de C. parvum ou de ses acides polynucléiques spécifiques.

22. Amorce de réaction de polymérisation en chaîne consistant en un oligonucléotide ayant une séquence d'extrémité 5' de SEQ ID N° 3, 4, 9 ou 10.

23. Amorce suivant la revendication 22, consistant en un oligonucléotide de séquence SEQ ID N° 3, 4, 9 ou 10.

24. Méthode suivant l'une quelconque des revendications précédentes, comprenant un mode opératoire pour la libération d'acides nucléiques de l'organisme en question avant l'entrée en vigueur des conditions de réaction en chaîne d'amplification.

25. Méthode suivant la revendication 24, dans laquelle l'échantillon à étudier est exposé aux ultrasons ou soumis à des cycles de congélation/décongélation en vue de rompre les organismes et de libérer les acides nucléiques.

26. Méthode suivant la revendication 24 ou 25, dans laquelle les organismes sont tout d'abord mis en suspension dans un milieu.

27. Méthode suivant l'une quelconque des revendications 24 à 26, dans laquelle les organismes sont sous la forme d'oocystes.

28. Sonde suivant la revendication 3, 14 ou 15, dans laquelle l'oligonucléotide est marqué par incorporation d'un marqueur radioactif, chimique ou biologique à sa structure.

29. Kit diagnostique comprenant une ou plusieurs sondes ou amorces suivant l'une quelconque des revendications 3, 7, 8, 14, 15, 22, 23 ou 28.

30. Kit diagnostique suivant la revendication 29, comprenant en outre des anticorps marqués par un métal contre les oocystes de Cryptosporidium.
